# EUROPEAN PATENT APPLICATION

(11) **EP 1 742 057 A1**
(43) Date of publication of application: **10.01.2007**
(21) Application number: 05076569.2
(22) Date of filing: 08.07.2005
(51) Int. Cl.: G01N 33/543, G01N 33/68

(54) **Device and method for the separation of particles**

(71) Applicant: Stichting voor de Technische Wetenschappen, 3527 JP Utrecht (NL)
(72) Inventor: Kohlheyer, Dietrich, 7523 ZT Enschede (NL); Schasfoort, Richardus Bernardus Maria, 7571 AJ Oldenzaal (NL)
(74) Representative: Winckels, Johannes Hubertus F.

(57) **Abstract**

A device for separating, analysing, detecting and/or determining particles in a liquid sample flow, particularly a microchip (1), wherein the device (1) comprises:
- at least one sample flow separation area (4);
- a sample flow supply (2) to supply the sample flow (S) to said sample flow separation area (4); and
- at least one field generator (5, 6) which is configured to generate a field to substantially laterally separate certain sample flow particles when the sample flow flows through said separation area (4), for example via separation by free flow electrophoresis, free flow isoelectric focusing and/or isotachophoresis ; wherein the device is provided with an at least 2-dimensional detection pattern (9) located at least partly in and/or downstream with respect to said separation area (4), wherein said detection pattern (9) is suitable to interact with certain sample flow particles to be detected and/or determined.

The invention also provides a method for separating, analysing, detecting and/or determining particles in a liquid sample flow.

## Description

The invention relates to a device and method for separating, analysing, detecting and/or determining particles in a liquid sample flow.

Various such devices and methods are known from the prior art. For example, WO 2004/109271 (Manz et al.) discloses a device and method for free flow electrophoresis (FFE) having a microchip comprising: a separation chamber, a plurality of inlet and outlet channels, and a magnetic field unit for providing a magnetic field across said separation chamber. During use, charged components introduced in the separation chamber can be deflected laterally. The separation system of the known device comprises a detection unit for detecting components driven through each of the outlet channels, to enable counting of the numbers of separated components. Downstream with respect to the detection unit, a plurality of collection units can be located, to collect separated components for further use.

A disadvantage of the known device and respective method is, that they are only suitable to detect a limited amount of particles. For example, in the field of Proteomics, it is desired to separate, analyse, detect and/or determine a large number of different particles, wherein for example the composition of many of the particles is yet unknown. The known method and device are particularly not well suited to discover, detect and/or separate a large number unknown particles in a desired relatively short period of time.

The present invention aims to solve the above-mentioned problems. Particularly, the present invention aims to provide a device and a method, capable to be used in separating, analysing, discovering, detecting and/or determining large numbers of particles in a liquid sample flow, in a relatively short timeframe.

According to an aspect of the invention, there is provided a device for separating, analysing, detecting and/or determining particles in a liquid sample flow, particularly a microchip, wherein the device comprises:
- at least one sample flow separation area;
- a sample flow supply to supply the sample flow to said sample flow separation area; and
- at least one field generator which is configured to generate a field to substantially laterally separate certain sample flow particles when the sample flow flows through said separation area, for example via separation by free flow zone electrophoresis, free flow isoelectric focusing and/or isotachophoresis;
wherein the device is provided with an at least 2-dimensional detection pattern located at least partly in and/or downstream with respect to said separation area, wherein said detection pattern is suitable to interact with certain sample flow particles to be detected and/or determined.

The device according to the invention can be used in separating, analysing, detecting and/or determining large numbers of particles in a liquid sample flow, in a relatively short timeframe. Particularly, the device can separate said sample flow first, for example in a number of flow parts of differently charged components, depending amongst others on the field to be used. Separated particle flows can then be fed along said at least 2-dimensional detection pattern, for example to further unravel the composition of the separated flow parts. For example, the invention can provide a coupling between separation of sample flow particles and detection of sample flow particles. To this aim, for example, the at least 2-dimensional detection pattern can extend substantially parallel to an average flow direction of the (separated) sample flow which can flow along the detection pattern during use.

For example, the at least 2-dimensional detection pattern can serve to obtain a certain 'fingerprint' of the sample flow. Such a 'fingerprint' can be used, for example, to determine and/or analyse one or more of the sample flow components, to discover new particles, yet unknown, to continuously analyse a composition of the sample flow, for example to detect changes in that composition real-time, and/or to be used for other purposes.

Advantageously, said detection pattern can include at least a first detection area and at least a subsequent second detection area, which is located downstream with respect to said first detection area, wherein preferably each detection area is configured to interact with one specific particle, or one type of particles, only. In this way, the fingerprinting of the sample flow can be achieved in a reliable manner. As an example only, a device according to the invention may be arranged to separate a sample flow first in a separation area on charge (for example the following five charge types --, -, 0, +, ++). Then, different subsequent detection areas can be configured to subsequently detect different particles, or particle types (for example four types A, B, C, D). In such an exemplary embodiment, at least 20 different types of components of the sample flow (namely 5 charge groups of particles --, -, 0, +, ++ times four particle types A-D) can be separated, detected, analysed and/or determined.

Alternatively, the detection areas can each be configured to interact with a plurality of particles, or types of particles. In this way, an even larger amount of different particles can be separated, detected, analysed and/or determined.

For example, said at least subsequent second detection area can be configured to interact with one or more different particles than said first detection area.

Also, preferably, said detection pattern can include a number of elongated, substantially uninterrupted detection areas, each detection area preferably extending substantially laterally with respect to an average flow direction of sample flow, or part thereof, which flows along the detection area during use. In an embodiment of the invention, the detection pattern can include an array of substantially parallel, preferably elongated, detection areas.

In this way, preferably, all parts of a sample flow, being separated in a separation area, can be fed along the detection pattern, so that certain separated sample flow particles can to interact with the detection pattern. For example, in this way, a sample flow can be substantially continuously 'fingerprinted' for a very large number of different particles and/or particle types, after having been separated in one or more separation areas. Preferably, to this aim, the width of said separation area, measured laterally with respect to an average flow direction of sample flow flowing through that area, is about the same as a width of said detection pattern, measured laterally with respect of sample flow flowing along the detection pattern.

Various types of fields can be used to separate the sample flow. For example, electromagnetic fields can be used, for example to achieve via separation by free flow electrophoresis, free flow isoelectric focusing and/or isotachophoresis.

In case said field generator is an electrical field generator, including a number of electrodes arranged near said separation area, preferably a hydrogel compound is arranged between each said electrodes and the separation area. It has been found that good separation results can be obtained in this way, wherein relatively low electrode potential differences can be utilized. An example of said hydrogel compound, in a different appliance and for a different purpose, can be found in Yuzuru Takamura et al., "Low-voltage electroosmosis pump for stand-alone microfluidics devices", Electrophoresis 2003, 24, 185-192.

In an embodiment of the invention, the device can be configured to establish a pH-gradient across at least part of said sample flow separation area, for example by supplying a fluid containing ampholites or related compounds to the separation area. In this way, iso-electric separation of the sample can be obtained.

Preferably, said detection pattern is configured to bind to certain particles to be detected, for example such that different particles can be bound to different locations of the detection pattern.. For example, said detection pattern can include a pattern of binding molecules, the binding molecules being suitable to bind to certain particles to be detected and/or determined when the sample flow, or part thereof, flows along the detection pattern. Also, at least one of said binding molecules can be a specific binding molecule suitable to bind to certain particles to be detected, preferably a member of a specific binding pair such as an antibody and/or its antigen or a receptor and/or its ligand. Besides, at least one of said specific binding molecules can be an antibody or a functional part, derivative and/or analogue thereof. Moreover, said functional part can be an antigen binding part of an antibody, preferably an Fab-fragment. Further, wherein said functional derivative can be a single chain variable fragment (ScFv) or a monobody. In an aspect of the invention, said analogue can be a binding body comprising at least one Ig-like fold of a non-antibody protein or a functional equivalent thereof. Also, a mixture of DNA fragments can be detected, and a specific ssDNA strands can be immobilised on the detection pattern. Besides, the detection pattern may be suitable to detect Polymerase chain reaction products. For example, first fragments can be separated on basepair length and at a detection area on the specific hybridisation on the fragments. For example, both receptor, ssDNA, ligands can be immobilized on the detection pattern.

Besides, the device can be arranged in various ways to detect, measure and/or analyse certain particles. For example, the device can be provided with and/or is arranged to cooperate with at least one detector, the detector being configured to detect whether certain particles and certain binding molecules of said pattern of binding molecules are bound to each other. Advantageously, surface plasmon resonance is used to detect interaction between said detection pattern and certain sample flow particles. On the other hand, fluorescence microscopy, mass spectrometry, laser technology and/or digital imaging technology, and/or other methods, can be utilized, see for example NL 1019446 and/or NL 1019875, depending amongst others on the particles to be detected and/or analyzed.

According to an aspect of the invention, there is provided a device for separating particles in a liquid sample flow, for example a device according to any of the preceding claims, particularly a microchip, wherein the device comprises a first and a second sample flow separation area, as well as a separator arranged between said first and second sample flow separation area, the separator being configured to separate sample flow received from the first sample flow separation area at least into one part which is passed onto said second sample flow separation area during use, and another part, wherein each separation area is preferably associated with a respective field generator configured to generate a field across at least part of the respective sample flow separation area, for example to substantially laterally separate certain sample flow particles when they move through that area, for example via separation by free flow electrophoresis, free flow isoelectric focusing and/or isotachophoresis.

In this way, a sample flow can be unraveled into various components with improved resolution. For example, at least second separation step can be carried out in said second sample flow control area, to further zoom-in or focus on part of the sample flow that was already separated in said first sample flow separation area.

In an other aspect of the invention, there is provided a device for separating particles in a liquid sample flow, for example a device according to any of the preceding claims, particularly a microchip, wherein the device at least comprises:
- at least one sample flow separation area;
- at least one field generator which is configured to generate a field, to substantially laterally separate sample flow, or certain sample flow particles, flowing through said separation area during use, for example via separation by free flow electrophoresis, free flow isoelectric focusing and/or isotachophoresis ;
- a separator arranged downstream with respect to said sample flow separation area, the separator being configured to separate at least one lateral part of the sample flow received from the sample flow separation area from a remaining sample flow part; and
- a control to provide lateral steering of the sample flow, or part thereof, with respect to said separation area during use, wherein the control is preferably provided with at least one steering fluid supply to supply steering fluid to said separation area to achieve hydrodynamic steering.

Thus, the sample flow can be separated laterally, wherein - preferably- one of the laterally separated sample flow parts can be directed towards the separator, to be separated from a remaining sample flow part, in an efficient continuous manner.

An aspect of the invention provides the use of a device according to the invention, for example to detect, determine, study and/or analyse proteins. This provides above-mentioned advantages, for example in the field of Proteomics.

Also, an aspect of the invention provides a method for separating, analysing, detecting and/or determining particles, for example proteins, in a liquid sample flow, particularly carried out by a device according to the invention, wherein the method includes:
- supplying a sample flow to at least one sample flow separation area; and
- generating at least one field in/across at least part of said sample flow separation area, to substantially laterally separate certain sample flow particles, for example via separation by free flow electrophoresis, free flow isoelectric focusing and/or isotachophoresis ;
wherein said sample flow, or part thereof, is being fed along an at least 2-dimensional detection pattern suitable to interact with certain sample flow particles to be detected and/or determined. This can provide above-mentioned advantages.

Besides, an aspect of the invention provides a method for separating particles, for example proteins, in a liquid sample flow, wherein the method includes:
- supplying a sample flow to at least a first sample flow separation area; and
- generating at least one field across at least part of said sample flow separation area, to substantially laterally separate the sample flow, or certain sample flow particles, for example via separation by free flow electrophoresis, free flow isoelectric focusing and/or isotachophoresis ;
wherein part of the sample flow, which part has been separated in the first sample flow separation area from a remaining sample flow part, is being passed to at least one second sample flow separation area, to be separated further in the second sample flow separation are, wherein preferably at least one field is being generated across at least part of said second sample flow separation area, to laterally separate the sample flow part, or certain sample flow particles, flowing through the second sample flow area, for example via separation by free flow electrophoresis, free flow isoelectric focusing and/or isotachophoresis .

In an other aspect of the invention there is provided a method for separating particles, for example proteins, in a liquid sample flow, wherein the method at least includes:
- supplying a sample flow to a first sample flow separation area ;
- generating at least one field across at least part of said sample flow separation area, to substantially laterally separate certain sample flow particles, for example via separation by free flow electrophoresis, free flow isoelectric focusing and/or isotachophoresis ; and
- steering a laterally separated sample flow part, for example towards a sample flow collector and/or sample flow separator. The steering is preferably carried out by hydrodynamic steering. An example of hydrodynamic steering as such, used in hydrodynamic focusing, can be found, for example, in WO 00/56444 (Blankenstein et al.).

Embodiments of the invention will now be described, by way of example only, with reference to the accompanying schematic drawings in which corresponding reference symbols indicate corresponding parts, and in which:
figure 1 schematically depicts a first embodiment of the invention;
figure 2 schematically depicts a second embodiment of the invention;
figure 3 schematically depicts a third embodiment of the invention;
figure 4 schematically depicts a fourth embodiment of the invention;
figures 5A-5C schematically depict a fifth embodiment of the invention, in various steering modes;
figures 6A-6B schematically depict a sixth embodiment of the invention, in two alternative steering modes;
figures 7A-7C depict a seventh embodiment of the invention, in three different modes;
and Fig. 8 depicts a result of an embodiment of the invention.

In the present application, corresponding or similar features are denoted by corresponding or similar reference signs.

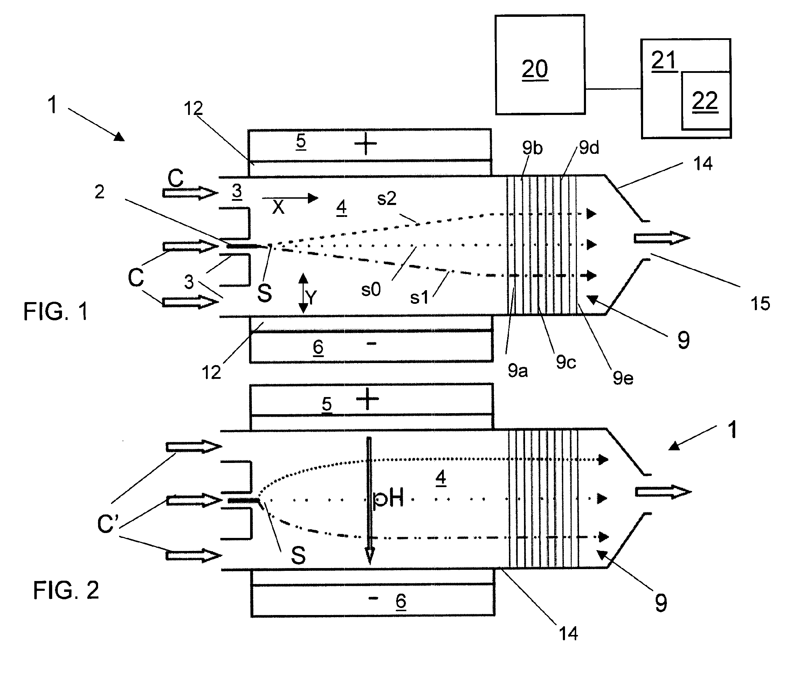

Figure 1 shows an embodiment of a device for separating, analysing, detecting and/or determining particles in a liquid sample flow S. The sample flow S can contain a sample, for example, which contains fluid emanating from a living species, for example a human or animal body, and/or a different sample.

The device is preferably arranged in the form of a microchip 1, or part thereof. The device 1 comprises a sample flow supply 2 to supply a sample flow S to a sample flow separation area 4 of the device 1. Said sample flow supply 2 can be arranged in various ways, as will be clear to the skilled person. In the present embodiment, the sample flow supply 2 is arranged to supply the sample flow S substantially to a centre line of the separation area 4; however, alternatively, one or more sample flow supplies can be arranged to supply sample flow(s) to different locations of the separation area 4. In the present embodiment, the device 1 also includes further fluid supplies 3 to supply other fluids C to the separation area 4, for example carrier fluids C which can encompass said sample flow S, and which might be used to actively, for example hydrodynamically, steer a sample flow S in a lateral direction, for example to laterally position the sample flow S with respect to the separation area 4 and/or a downstream part of the device 1.During use, the said sample flow S and optional carrier flows C are substantially laminar flows in the separation area, as will be clear to the skilled person.

Besides, the device 1 can include one or more reservoirs (not shown) to contain one or more samples to be separates and/or analysed. Similar reservoirs (not shown) may be included to contain sample carrier fluids. Also, the device 1 can include one or more pumps (not shown) to continuously pump a sample, as sample flow S, to said flow separation area 4. The initiating of the flowing of the sample flow S can be achieved in various ways, which will be clear to the skilled person. The chamber 14 also includes an outlet 15 downstream with respect to separation area.

Said sample flow separation area 4 can be configured in various ways. For example, the separation area 4 can be located in a suitable chamber 14, through which the sample flow S can be fed. Preferably, the dimensions of the sample flow separation area are relatively small, which can be achieved, for example, when the device is a microchip 1.

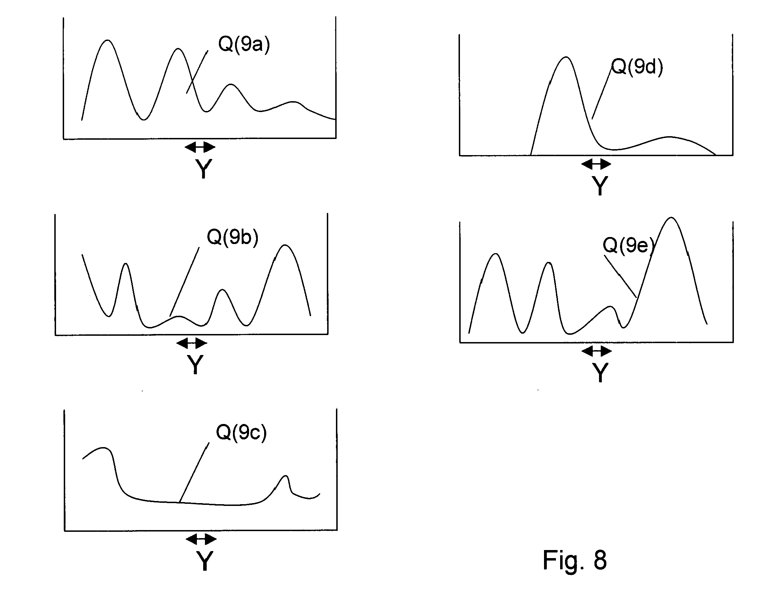

As is shown in Fig. 1, the device 1 can be provided with an electromagnetic field generator, including electrodes 5, 6 located on opposite sides of the separation area 4, to generate an electromagnetic field across most of said sample flow separation area 4. In the present embodiment, a hydrogel compound 12 is arranged between each of said electrodes and the separation area 4, to electrically bridge or join the content of the separation area 4 with said electrodes 5, 6 during use. The electrodes 4, 5 can be connected to an electrical power supply (not shown) to generate an electric field across the separation area. Thus, the electrodes 4, 5 can be used to achieve a substantially lateral separation of charged components of the sample flow S, for example via separation by free flow electrophoresis, free flow isoelectric focusing and/or isotachophoresis. In figures 1 and 8, double arrow Y indicates lateral directions and arrow X an average sample flow direction X, of the sample flow S flowing through the chamber 14.

The first embodiment 1 is further provided with a 2-dimensional detection pattern 9 suitable to interact with certain particles to be detected and/or determined. In the present embodiment, the 2-dimensional detection pattern 9 is located between said separation area 4 and the outlet 15, such that said sample flow S, or laterally separated parts s0, s1, s2 thereof, can be fed from the separation area 4 along the detection pattern 9 to the outlet 15 during use. Alternatively, the detection pattern 9 may also extend, for example, at least partly in the separation area 4 of the chamber 14.

The detection pattern 9 can extend on and/or be provided in, for example, a surface of the device 1, such that said sample flow S can be directed along the surface and the respective pattern 9. In the present embodiment, the 2-dimensional detection pattern 9 extends along a two-dimensional (virtual) plane which is substantially parallel to the average flow direction X of the sample flow S, see Fig. 1. Particularly, the pattern 9 includes an array of substantially parallel, elongated detection areas 9a, 9b, 9c, 9d, 9e, located at small distances from each other. The detection areas 9a-9e each extend laterally (or perpendicularly) with respect to said average flow direction X, and transversally with respect to the chamber 14. Preferably, each detection area 9a-9e is configured to interact with one specific particle, or one type of particles, only. For example, the upstream detection area 9a can be configured to interact with a first specific particle A, or type of particles A, the second detection area 9b can be configured to interact with a second specific particle B, or type of particles B, and so on. Preferably, all of the detection areas are configured to interact with respective different particles. Therefore, during use, different particles can be bound to different locations of the detection pattern 9.

Preferably, each elongated detection area 9a-9e is substantially uninterrupted, viewed in longitudinal direction of that detection area. Also, in the present embodiment, the width of said separation area 4, measured laterally with respect to the average flow direction X of the sample flow S, is about the same as a width of said detection pattern 9, measured in the same direction, see fig. 1.

In a further aspect of the invention, as has been mentioned above, said detection pattern can be, or include, an at least 2-dimensional pattern of binding molecules 9, the binding molecules being suitable to interact with certain particles to be detected and/or determined by binding to those particles. For example. at least one of said binding molecules can be a specific binding molecule suitable to bind to certain particles to be detected, preferably a member of a specific binding pair such as an antibody and/or its antigen or a receptor and/or its ligand. For example, each detection area 9a-9e of the detection pattern 9 can include a substantially uninterrupted, elongated row and/or elongated layer of binding molecules suitable to bind to certain particles to be detected and/or determined.

The first embodiment 1 is preferably provided with and/or is arranged to cooperate with at least one detector 20, or sensor, the detector 20 being configured to detect whether certain particles and certain binding molecules of the 2-dimensional pattern of binding molecules 9 are bound to each other. Such a detector 20 can be configured in various ways, depending amongst others on the particles to be detected and/or on the configuration of the detection pattern.

For example, said detector 20 can include a surface plasmon resonance detector, a fluorescence microscope, mass spectrometer, laser detector, or digital imaging detector, and/or other detectors. The detector can be suitable to be used in acoustic detection, optical detection, electrochemical detection, DNA hybridisation reaction detection, amperometry, potentiometry, conductometry and/or detection techniques. In an embodiment of the invention, the detector includes a Magnetoresistive (GMR) device, for example to detect magnetic particles. Detection of certain particles can be carried out using various labeling-techniques, for example fluorescence labeling, chemoluminescent labeling, radioactive labeling and/or other labeling methods. The detector 20 may be configured to use a particle detection technique wherein a surface is being used. For example, suitable detection methods can include attenuated total reflection, interferometry, wave guides and/or other techniques wherein evanescent fields are utilized. Besides, more selective devices or methods can be used, wherein a coupling or interaction takes place with/as a result of a reaction, for example enzyme sensors. For example, one ore more lanes of enzymes can be provided or laid down on an array of electrochemical detectors.

The detector 20 or respective particle detection method can include one or more of the mentioned detectors or methods, and/or other detectors or methods, as will be clear to the skilled person. The device of figure 1 can be used to detect and/or determine a large number particles in a liquid flow, for example to detect, determine, study and/or analyse proteins. During use, the sample flow supply 2 can feed a sample flow S to the sample flow separation area 4. Preferably, carrier flows C are introduced into the separation area as well. An electrical field can be generated across the sample flow separation area 4, utilizing the electrodes 5, 6. Depending on the composition of the sample flow S, the field can separate one or more parts of the sample flow S substantially laterally, for example via separation by free flow electrophoresis. The amount of lateral separation can depend, for example, on the field strength, the composition of the sample flow, the masses of the respective particles, the average flow rate and/or the like.

Resulting separated sample flow parts, only three of which are shown by dashed lines s0, s1, s2 in Fig. 1, can then be fed along the 2-dimensional detection pattern 9a-9e, downstream with respect to the separation area 4. Depending on the composition of the sample flow parts s0, s1, s2, certain sample flow particles can bind to binding molecules of the detection pattern 9a-9e, on various respective lateral locations. Such interaction is preferably detected by said detector 20, for example real-time, to be processed by a suitable data processor 21 and/or to be stored in a suitable memory 22.

This can result in a sort of fingerprinting of the sample flow S. Fig. 8 shows a possible result that might be obtained by using the device 1 of Fig. 1. Figure 8 depicts five different detection results (for example detector signals or detector signal related data) Q(9a), Q(9b), Q(9c), Q(9e), as function of said lateral direction Y, relating to respective detection of particle interaction with the respective five different elongated detection areas 9a-9e. The results can depend, amongst others, on the composition of the sample flow S, the separation method used in the sample flow area 4 (for example Free Flow Electrophoresis) and the composition of each of the detection lanes, or areas, 9a-9e of the detection pattern 9. For example, the graphs Q(9a), Q(9b), Q(9c), Q(9e) of Fig. 8 are spectra resulting from interactions between sample flow components and the elongated detection areas 9a-9e. Preferably, changes in the composition of the sample flow S can be detected and analysed real time, for example by the data processor 21. The 'fingerprint' type, sample flow dependent result shown in Fig. 8 can be used, for example, to monitor a sample of a fluid of a patient, such as blood or sweat, real-time, so that changes of a certain condition of the patient can be detected swiftly. For example, a substantially complete fingerprint of a sample containing a protein-mixture can be obtained in this way, depending on the arrangement of the detection pattern 9.

The embodiment of Figure 2 differs from the embodiment shown in Fig. 1, in that the device 1 is configured to establish a pH-gradient across at least part of said first sample flow separation area 4 during use, particularly to achieve iso-electric lateral separation of the sample flow S during use. For example, the device can be configured to supply a carrier fluid C' to the separation area 4, which carrier fluid C' contains a compound for establishing a pH-gradient in the electromagnetic field generated across the separation area 4 during use. To this aim, the carrier fluid can comprise for example ampholites (see WO 2004/109271, or Yi Xu et al., "Sub-second isoelectric focusing in free flow using a microfluidic. Device, Lab Chip, 2003, 3, 224-227).

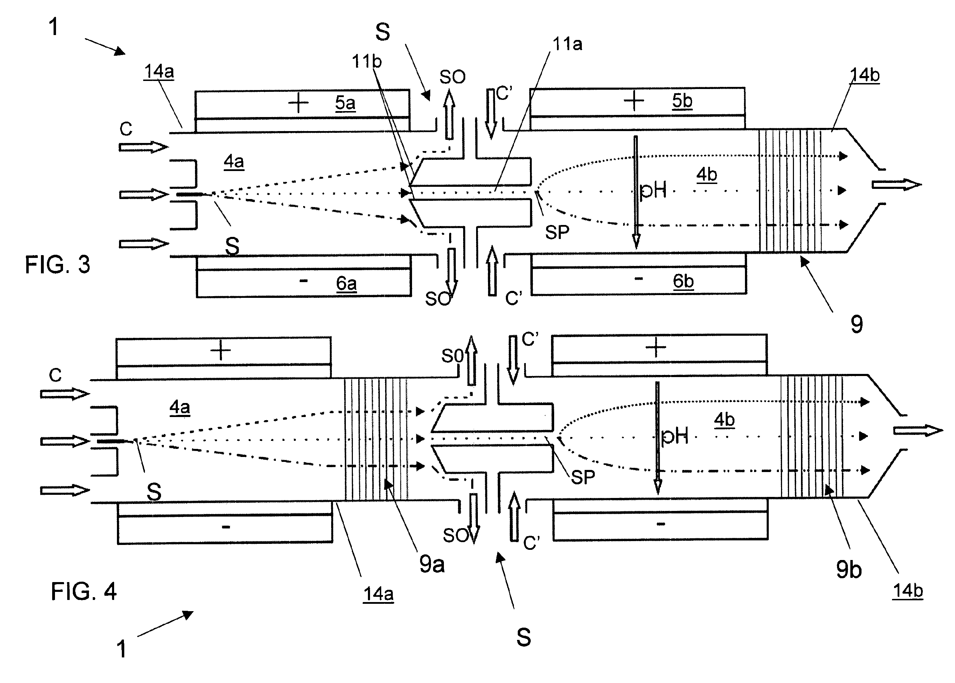

Figure 3 depicts a third embodiment of the invention. The third embodiment differs from the first embodiment, in that the device 1 of fig. 3 includes a first sample flow separation area 4a, in a first chamber 14a, and a second sample flow separation area 4b, in a second chamber 14b, as well as a separator 11 arranged between said first sample flow separation area 4a and second sample flow separation area 4b. The separator 11 can be configured to separate sample flow S received from the first sample flow separation area 4a at least into one part SP which is passed onto said second sample flow separation area 4b during use, and another part SO, which can be passed to another part of the device 1. The separator 11 as such can be configured in various ways. In the present embodiment, for example, the separator 11 simply includes a small central channel 11a, extending between the first and second chamber 14a, 14b, for example centrally with respect to centre axes of the chambers 14a, 14b. The separator can also provide outlet channels 11b to drain remaining sample flow parts SO. The separator 11 can include relatively sharp separation tips or side walls 11b, directed towards the first separation area 4a, to abruptly separate incoming sample flow into desired parts SP, SO.

Both separation areas 4a, 4b of the third embodiment are provided with respective electrodes 5a, 6a, 5b, 6b, to generate electric fields across these areas 4a, 4b. In the third embodiment, a detection pattern 9, for example comprising suitable binding molecules, is located downstream with respect to said second separation area 4b. As an example, the first separation area can be used to separate incoming sample flow S by Free Flow Electrophoresis. One of the resulting, laterally separated sample flow parts SP can then be separated by the separator 11 from a remaining sample flow part SO, to be further separated in the second separation area 4b, for example via free flow isoelectric focusing. The resulting sample flow part, that has been separated laterally various times using different methods, can then be fed along the detection pattern 9, so that certain molecules of the resulting sample flow part SP may interact with binding molecules of that pattern, as has been described above.

Figure 4 depicts a fourth embodiment of the invention. The fourth embodiment differs from the third embodiment, in that the device 1 of fig. 4 also includes a detection pattern 9a located between the separator 11 and the first sample flow separation area 4a. In this ways, results of the detection patterns 9a, 9b of both chambers 14a, 14b may be combined for obtaining more data to analyse the composition of the sample S.

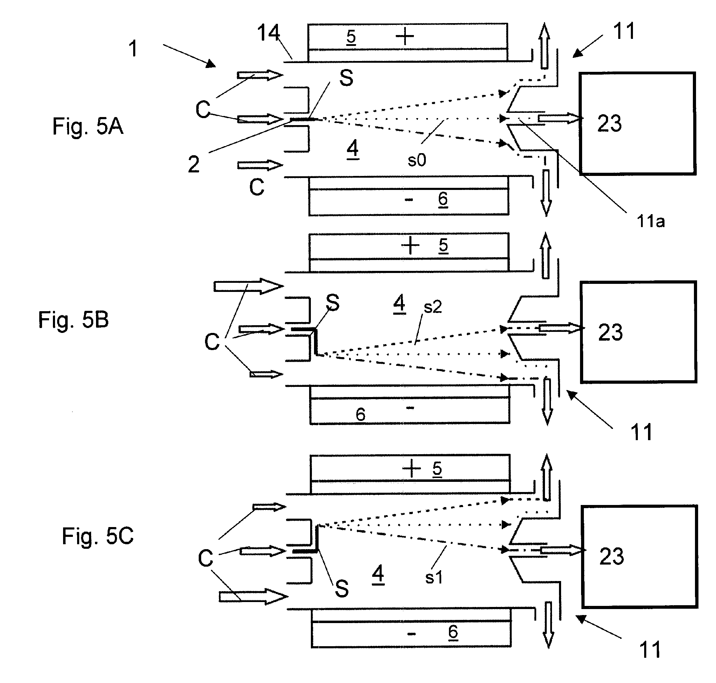

Figure 5 depicts a fifth embodiment of the invention. The fifth embodiment can be used, for example, in combination with one or more of the above-described embodiments of Figures 1-4, or as part of one of those embodiments. The embodiment of Fig. 5 differs from the first embodiment in that the chamber 5 is not particularly provided with a detection pattern 9. In Fig. 5, the device 1 includes: a sample flow separation area 4, a sample flow supply 2 to supply the sample flow S to said sample flow separation area 4, as well as electrodes 5, to generate a field to substantially laterally separate certain sample flow particles when the sample flow flows through said separation area 4. The fifth embodiment is further arranged to provide a lateral steering of the sample flow S, or parts thereof, preferably utilising hydrodynamic steering. Besides, the fifth embodiment is provided with a separator 11, the construction of which can be substantially the same as the separator construction of the third embodiment (see above and Fig. 3).

Particularly, the fifth embodiment is provided with a control to laterally position the sample flow S, or part thereof, with respect to the separation area 4 during use, wherein the control provided with steering fluid supplies to supply steering fluid C to said separation area 4 to achieve hydrodynamic steering.

During use of the fifth embodiment, separating particles, for example proteins, of a liquid sample flow can be separated, wherein the sample flow S is being supplied to the sample flow separation area 4; -generating at least one field across at least part of said sample flow separation area 4, to substantially laterally separate certain sample flow particles , for example via separation by free flow electrophoresis; and - steering a laterally separated sample flow part, for example towards a sample flow collector 23 and/or sample flow separator, utilising the steering flows C. For example, Fig. 5A depicts how a central sample flow part s0 is being actively steered into the central channel 11a of the separator 11. In Fig. 5B, the flow rates of the steering fluids C have been adjusted such, that another lateral sample flow part s2 enters the central channel 11a of the separator 11. In Fig. 5C, the steering fluids have been configured to actively direct yet another laterally separated sample flow part s1 into the central separator channel 11a. For example, the steering fluids C can be adjusted such, that the laterally separated sample flow is scanned laterally with respect to the separator 11, so that subsequent lateral parts of that sample flow S can be directed into the separation channel 11a one after another.

Downstream with respect to the separator 11, for example, one or more sample collectors 23, sample analysing means and/or particle detection patterns can be located (not shown in Fig. 5), to collect sample parts, analyse sample parts and/or detect sample parts. Also, in the fifth embodiment, a said detection pattern 9 may be located between the separator 11 and separation area 4 (a in Fig. 4).

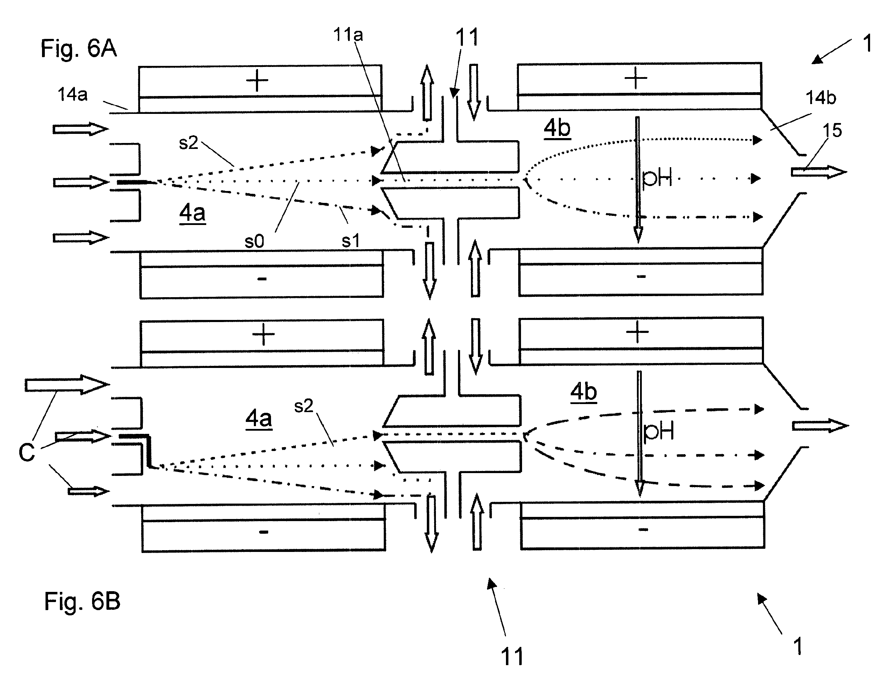

Figures 6A and 6B show a further embodiment of the invention, which is similar to the embodiment shown in Fig. 3. However, the embodiment shown in Figures 6A, 6B does not have to be provided with said detection pattern 9. In Figures 6A, 6B, active, hydrodynamic steering is applied to sample flow S flowing through the first separation area, similar to the above-described steering in the fifth embodiment. For example, a laterally separated part s0, s1, s2 of the sample flow S can be directed into the separation channel 11a of the separator, to be fed to the second separation area 4b, and to be further separated laterally in the second separation area 4b. In Fig. 6, for example, free flow electrophoresis is applied in the first separation area 4a, and free flow isoelectric focusing in the second separation area 4b. However, different field dependent separation methods can also be applied. For example, free flow electrophoresis, free flow isoelectric focusing or isotachophoresis can be applied in each of the subsequent separation areas 4a, 4b. In this way, the device can 'zoom in' or focus onto a certain part of the sample S, particularly real-time. Although not show, the embodiment of Figures 6A, 6B can also be provided with one or more said detection 2-dimensional patterns 9, to detect and/or analyse certain sample flow particles.

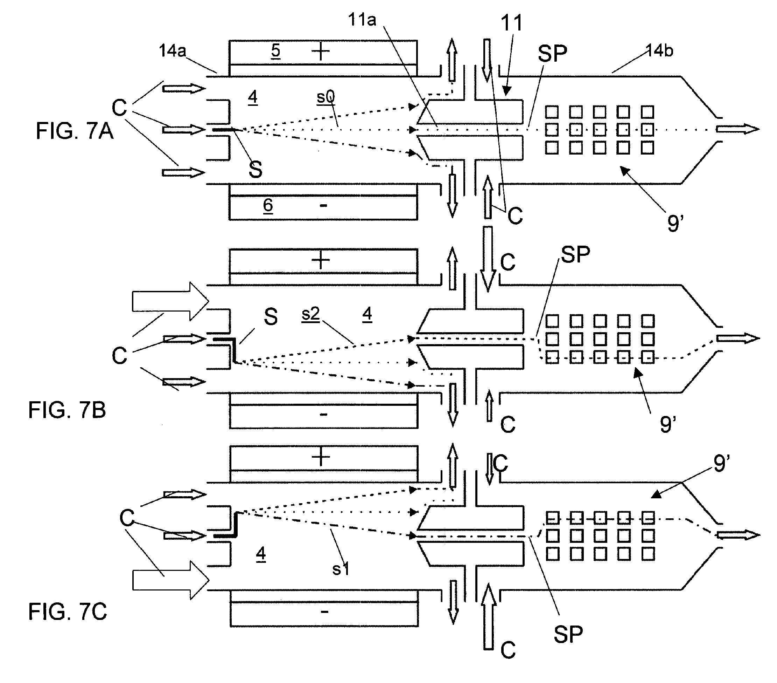

Figures 7A-7C depict a seventh embodiment of the invention. The seventh embodiment differs from the fifth embodiment (see Fig. 5), in that a second chamber 14b is provided, downstream with respect to the separator 11, wherein the second chamber is provided with a 2-dimensional detection pattern 9', for example including a grid of spaced-apart binding sites. As in the fifth embodiment, in the seventh embodiment, the sample flow S can be steered hydrodynamically with respect to the separation area 4 of the first chamber 14a. Besides, a control is provided to laterally position sample flow SP, flowing from the separator 11 into the second chamber 4b, towards a desired part of the detection pattern 9'. As is shown in Figures 7A-7C, the lastmentioned control can also include hydrodynamical steering of said sample flow part SP.

The descriptions above are intended to be illustrative, not limiting. Thus, it will be apparent to one skilled in the art that modifications may be made to the invention as described without departing from the scope of the claims set out below.

For example, the particles to be separated, analysed, detected and/or determined can include certain molecules, proteins, marked particles, cells, cell fractions and/or other particles.

Besides, a said detection pattern may include a 2-dimensional or 3-dimensional detection pattern. As an example, a sample S can be separated in different lateral directions in a separation area, for example using different fields.

## Claims

1. A device for separating, analysing, detecting and/or determining particles in a liquid sample flow, particularly a microchip (1), wherein the device (1) comprises:
- at least one sample flow separation area (4);
- a sample flow supply (2) to supply the sample flow (S) to said sample flow separation area (4); and
- at least one field generator (5, 6) which is configured to generate a field to substantially laterally separate certain sample flow particles when the sample flow flows through said separation area (4), for example via separation by free flow electrophoresis, free flow isoelectric focusing and/or isotachophoresis ;
wherein the device is provided with an at least 2-dimensional detection pattern (9) located at least partly in and/or downstream with respect to said separation area (4), wherein said detection pattern (9) is suitable to interact with certain sample flow particles to be detected and/or determined.

2. A device according to claim 1, wherein said 2-dimensional detection pattern (9) is configured to bind to certain sample flow particles when said sample flow, or part thereof, flows along the 2-dimensional detection pattern.

3. A device according to claim 1or 2, wherein said detection pattern (9) includes at least a first detection area (9a) and at least a subsequent second detection area (9b-9e), downstream with respect to said first detection area (9a), , wherein preferably each detection area is configured to interact with one specific particle, or one type of particles, only.

4. A device according to any of the preceding claims, wherein said detection pattern (9) includes a number of elongated, substantially uninterrupted detection areas, each detection area extending substantially laterally with respect to an average flow direction (X) of said sample flow (S), or part thereof, flowing there-along during use.

5. A device according to any of the preceding claims, wherein said separation area and said detection pattern are located substantially next to each other, or near one another, for example in a flow chamber.

6. A device according to any of the preceding claims, wherein said field generator is an electrical field generator (5, 6) which includes a number of electrodes (5, 6) arranged near said separation area (4), wherein preferably a hydrogel compound is arranged between each said electrodes and the separation area (4).

7. A device according to any of the preceding claims, wherein said detection pattern includes a pattern of binding molecules (9), the binding molecules being suitable to bind to certain particles to be detected and/or determined.

8. A device according to claim 7, wherein at least one of said binding molecules is a specific binding molecule suitable to bind to certain particles to be detected, preferably a member of a specific binding pair such as an antibody and/or its antigen or a receptor and/or its ligand.

9. A device according to claim 7 or 8, wherein the device (1) is provided with and/or is arranged to cooperate with at least one detector (20), the detector (20) being configured to detect whether certain particles and certain binding molecules of said pattern of binding molecules (9) are bound to each other.

10. A device according to any of the preceding claims, including at least a first sample flow separation area (4a) and a second sample flow separation area (4b), as well as a separator (11) arranged between said first sample flow separation area (4a) and second sample flow separation area (4b), the separator (1) being configured to separate sample flow (S) received from the first sample flow separation area (4a) at least into one part which is passed onto said second sample flow separation area (4b) during use, and another part, wherein preferably said detection pattern (9) is located at least partly in and/or downstream with respect to said second separation area (4b).

11. A device according to any of the preceding claims, including a control to laterally position the sample flow (S), or part thereof, with respect to the separation area (4) during use, wherein the control is preferably provided with at least one steering fluid supply (3) to supply steering fluid (C) to said separation area (4) to achieve hydrodynamic steering.

12. A device for separating particles in a liquid sample flow, for example a device according to any of the preceding claims, particularly a microchip (1), wherein the device (1) comprises a first sample flow separation area (4a) and a second sample flow separation area (4b), as well as a separator (11) arranged between said first sample flow separation area (4a) and second sample flow separation area (4b), the separator (1) being configured to separate sample flow (S) received from the first sample flow separation area (4a) at least into one part which is passed onto said second sample flow separation area (4b) during use, and another part, wherein each separation area (4a, 4b) is associated with a respective field generator (5, 6) configured to generate a field across at least part of the respective sample flow separation area (4), to substantially laterally separate certain sample flow particles when they move through that area (4), for example via separation by free flow electrophoresis, free flow isoelectric focusing and/or isotachophoresis.

13. A device for separating particles in a liquid sample flow, for example a device according to any of the preceding claims, particularly a microchip (1), wherein the device (1) includes:
- at least one sample flow separation area (4);
- at least one field generator (5, 6) which is configured to generate a field, to substantially laterally separate the sample flow, or certain sample flow particles, flowing through said separation area during use, for example via separation by free flow electrophoresis, free flow isoelectric focusing and/or isotachophoresis ;
- a separator (11) arranged downstream with respect to said (4) sample flow separation area (4), the separator (1) being configured to separate at least one lateral part of the sample flow (S) received from the sample flow separation area (4) from a remaining sample flow part; and
- a control to provide lateral steering of the sample flow (S), or part thereof, with respect to said separation area (4) during use, wherein the control is preferably provided with at least one steering fluid supply (3) to supply steering fluid (C) to said separation area (4) to achieve hydrodynamic steering.

14. Use of a device according to any of the preceding claims to detect and/or determine particles in a liquid flow, for example to detect, determine, study and/or analyse proteins.

15. Method for separating, analysing, detecting and/or determining particles, for example proteins, in a liquid sample flow, particularly carried out by a device according to any of claims 1-13, wherein the method includes:
- supplying a sample flow (S) to at least one sample flow separation area (4); and
- generating at least one field across at least part of said sample flow separation area (4), to substantially laterally separate certain sample flow particles, for example via separation by free flow electrophoresis, free flow isoelectric focusing and/or isotachophoresis ;
wherein said sample flow (S), or part thereof, is being fed along an at least 2-dimensional detection pattern (9) suitable to interact with certain sample flow particles to be detected and/or determined

16. Method according to claim 15, wherein said sample flow (S), or part thereof, is being fed along at least two subsequent detection areas (9a-9e) of said 2-dimensional detection pattern (9), , wherein preferably said at least two subsequent detection areas are configured to interact with one or more different particles, and/or wherein preferably each said detection area (9a-9e) is an elongated, substantially uninterrupted detection area, which extends substantially laterally with respect to an average flow direction (X) of said sample flow (S), or part thereof, which flows along that detection area.

17. Method according to claim 15 or 16, wherein part of the sample flow (S), which part has been laterally separated in a first sample flow separation area (4a) from a remaining sample flow part, is being passed to a second sample flow separation area (4b), to be separated laterally further in the second sample flow separation are (4b), wherein preferably said at least 2-dimensional detection pattern (9) is located at least partly in and/or downstream of said second sample flow separation area (4b).

18. Method for separating particles, for example proteins, in a liquid sample flow, for example as part of a method according to any of claims 15-17,
wherein the method includes:
- supplying a sample flow (S) to at least a first sample flow separation area (4a); and
- generating at least one field across at least part of said sample flow separation area (4), to substantially laterally separate the sample flow, or certain sample flow particles, for example via separation by free flow electrophoresis, free flow isoelectric focusing and/or isotachophoresis ;
wherein part of the sample flow (S), which part has been laterally separated in the first sample flow separation area (4a) from a remaining sample flow part, is being passed to at least one second sample flow separation area (4b), to be separated further in the second sample flow separation are (4b), wherein preferably at least one field is being generated across at least part of said second sample flow separation area (4b), to substantially laterally separate the sample flow part, or certain sample flow particles, flowing through the second sample flow area, for example via separation by free flow electrophoresis, free flow isoelectric focusing and/or isotachophoresis .

19. Method according to any of claims 15-18, wherein the sample flow (S) is also actively, laterally steered through and/or towards a desired section of said separation area (4), for example by hydrodynamically steering the sample flow (S).

20. Method according to any of claims 15-19, wherein at least one separated sample flow part is being collected downstream with respect to said separation area (4), for example to be stored and/or analysed.

21. Method according to any of claims 15-20, wherein interaction between certain particles and said detection pattern (9) is being carried out substantially real-time.

22. Method for separating particles, for example proteins, in a liquid sample flow, wherein the method at least includes:
- supplying a sample flow (S) to a sample flow separation area (4);
- generating at least one field across at least part of said sample flow separation area (4), to substantially laterally separate certain sample flow particles, for example via separation by free flow electrophoresis, free flow isoelectric focusing and/or isotachophoresis ; and
- steering a laterally separated sample flow part, for example towards a sample flow collector and/or sample flow separator.

23. Method according to any of the claims 15-22, wherein the liquid sample contains fluid emanating from a living species.
